(19) Europäisches Patentamt
European Patent Office
Office européen des brevets

(11) Veröffentlichungsnummer: **0 280 991 A1**

# (12) EUROPÄISCHE PATENTANMELDUNG

(21) Anmeldenummer: **88102602.5**

(51) Int. Cl.⁴: **C07D 231/38**, A01N 43/56

(22) Anmeldetag: **23.02.88**

(30) Priorität: **05.03.87 DE 3706993**

(43) Veröffentlichungstag der Anmeldung: **07.09.88 Patentblatt 88/36**

(84) Benannte Vertragsstaaten: **BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Ooms, Pieter, Dr.**
**Doerperhofstrasse 16**
**D-4150 Krefeld(DE)**
Erfinder: **Becker, Benedikt, Dr.**
**Metzkausener Strasse 14**
**D-4020 Mettmann(DE)**

## (54) 5-Amino-3-halogenalkyl-1-aryl-pyrazole.

(57) Es werden neue 5-Amino-3-halogenalkyl-1-aryl-pyrazole der allgemeinen Formel (I)

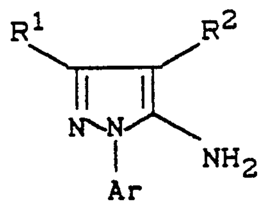

(I)

bereitgestellt, in welcher

$R^1$ für Halogenalkyl steht,

$R^2$ für Cyano, Hydroxycarbonyl, Carbamoyl, Thiocarbamoyl, Alkoxycarbonyl, N-Alkylcarbamoyl, N,N-Dialkylcarbamoyl, für Alkenyloxycarbonyl, N-Alkenylcarbamoyl, N,N-Dialkenylcarbamoyl, Alkinyloxycarbonyl, N-Alkinylcarbamoyl, N,N-Dialkinylcarbamoyl oder N-Arylcarbamoyl steht und

Ar für substituiertes Phenyl mit Ausnahme des 4-Nitrophenylrestes steht.

Die neuen Verbindungen der allgemeinen Formel (I) weisen eine starke insektizide Wirkung auf.

EP 0 280 991 A1

Xerox Copy Centre

# 5-Amino-3-halogenalkyl-1-aryl-pyrazole

Die Erfindung betrifft neue 5-Amino-3-halogenalkyl-1-aryl-pyrazole, mehrere Verfahren zu ihrer Herstellung und ihre Verwendung als Insektizide.

Es ist bereits bekannt, daß bestimmte 5-Amino-1-aryl-pyrazole, wie beispielweise das 4-Cyano-5-propionamido-1-(2,4,6-trichlorphenyl)-pyrazol oder das 4-Cyano-5-propionamido-1-(2,3,4-trichlorphenyl)-pyrazol gute herbizide Wirkung besitzen (vgl. z.B. DE-OS 32 26 513).

Weiterhin sind bestimmte 5-Amino-3-halogenalkyl-1-aryl-pyrazole, wie beispielsweise das 5-Amino-4-cyano-3-trifluormethyl-1-phenyl-pyrazol oder das 5-Amino-4-cyano-3-pentafluorethyl-1-phenyl-pyrazol oder das 5-Amino-4-cyano-3-heptafluorpropyl-1-phenyl-pyrazol oder das 5-Amino-4-methoxycarbonyl-3-trifluormethyl-1-phenyl-pyrazol oder das 5-Amino-4-carbamoyl-3-trifluromethyl-1-phenyl-pyrazol oder das 5-Amino-4-cyano-3-trifluormethyl-1-(4-nitro-phenyl)-pyrazol oder das 5-Amino-4-ethoxycarbonyl-3-trifluormethyl-1-phenyl-pyrazol oder das 5-Amino-4-methoxycarbonyl-3-trifluormethyl-1-(4-nitrophenyl)-pyrazol bekannt (vgl. Zh. org. Khim. 16, 1694 - 1698 [1980] bzw. C.A.: 94: 102 795 w; Zh. org. Khim. 17, 268-272 [1981] bzw. C. A.: 94: 17 39 714; Seikei Daigaku Kogakubu Hokuku 37, 2449-2450 [1984] bzw. C.A.: 101: 171 178 x ; J. Heterocyclic Chem. 23, 1535-1538 [1986]).

Über eine insektizide Wirksamkeit dieser vorbekannten Verbindungen ist bisher nichts bekannt.

Es wurden neue 5-Amino-3-halogenalkyl-1-aryl-pyrazole der allgemeinen Formel (I),

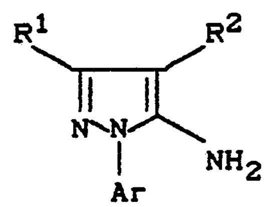

(I)

in welcher

$R^1$ für Halogenalkyl steht,

$R^2$ für Cyano, Hydroxycarbonyl, Carbamoyl, Thiocarbamoyl, Alkoxycarbonyl, N-Alkylcarbamoyl, N,N-Dialkylcarbamoyl, für Alkenyloxycarbonyl, N-Alkenylcarbomyl, N,N-Dialkenylcarbamoyl, Alkinyloxycarbonyl, N-Alkinylcarbamoyl, N,N-Dialkinylcarbamoyl oder N-Arylcarbamoyl steht und

Ar für substituiertes Phenyl mit Ausnahme des 4-Nitrophenylrestes steht;

gefunden.

Weiterhin wurde gefunden, daß man die neuen 5-Amino-3-halogenalkyl-1-aryl-pyrazole der allgemeinen Formel (I),

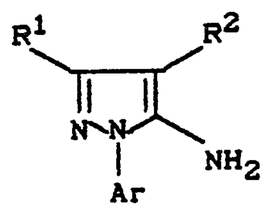

(I)

in welcher

$R^1$ für Halogenalkyl steht,

$R^2$ für Cyano, Hydroxycarbonyl, Carbamoyl, Thiocarbamoyl, Alkoxycarbonyl, N-Alkylcarbamoyl, N,N-Dialkylcarbamoyl für Alkenyloxycarbonyl, N-Alkenylcarbamoyl, N,N-Dialkenylcarbamoyl, für Alkinyloxycarbonyl, N-Alkinylcarbamoyl, N,N-Dialkinylcarbamoyl oder N-Arylcarbamoyl steht und

Ar für substituiertes Phenyl mit Ausnahme des 4-Nitrophenylrestes steht,

erhält, wenn man

(a) zum Erhalt von Verbindungen der Formel

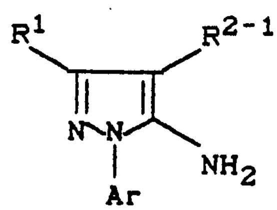

(Ia)

in welcher

$R^1$ und Ar die oben angegebene Bedeutung haben und

$R^{2-1}$ für Cyano, Carbamoyl, Thiocarbamoyl, Alkoxycarbonyl, N-Alkylcarbamoyl, N,N-Dialkylcarbamoyl, Alkenyloxycarbonyl, N-Alkenylcarbamoyl, N,N-Dialkenylcarbamoyl, Alkinyloxycarbonyl, N-Alkinylcarbamoyl, N,N-Dialkinylcarbamoyl oder für N-Arylcarbamoyl steht,

N-Arylhydrazid-halogenide der Formel (II),

$R^1$- C(Hal) = N-NH-Ar (II)

in welcher

$R^1$ und Ar die oben angegebene Bedeutung haben und Hal für Halogen steht,

mit Acetonitril-Derivaten der Formel (III),

$R^{2-1}$-$CH_2$-CN (III)

in welcher

$R^{2-1}$ die oben angegebene Bedeutung besitzt,

gegebenenfalls in Gegenwart eines Verdünnungsmittels und gegebenenfalls in Gegenwart eines Säurebindemittels umsetzt, oder wenn man

(b) zum Erhalt von Verbindungen der obengenannten Formel (Ia), in welcher $R^1$, $R^{2-1}$ und Ar die oben angegebene Bedeutung haben,

2-Chlor-acrylnitrilderivate der Formel (IV),

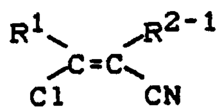

(IV)

in welcher

$R^1$ und $R^{2-1}$ die oben angegebene Bedeutung haben,

mit Arylhydrazinen der Formel (V),

Ar-NH-$NH_2$ (V)

in welcher

Ar die oben angegebene Bedeutung hat,

gegebenenfalls in Gegenwart eines Verdünnungsmittels und gegebenenfalls in Gegenwart eines Säurebindemittels umsetzt, oder wenn man

(c) zum Erhalt von Verbindungen der Formel

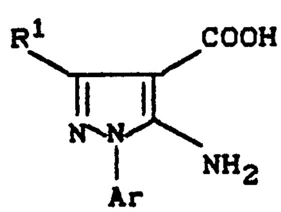

(Ib)

in welcher $R^1$ und Ar die oben angegebene Bedeutung besitzen,

die mit Hilfe der Verfahren (a) oder (b) erhältlichen 5-Amino-3-halogenalkyl-1-aryl-pyrazole der Formel (Ic),

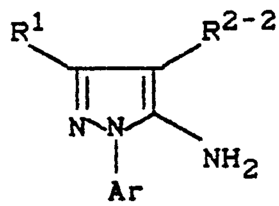

(Ic)

in welcher

R' und Ar die oben angegebene Bedeutung haben und

$R^{2-2}$ für Alkoxycarbonyl, Alkenyloxycarbonyl oder Alkinyloxycarbonyl steht,

mit Säuren gegebenenfalls in Gegenwart eines Verdünnungsmittels verseift.

Schließlich wurde gefunden, daß die neuen 5-Amino-3-halogenalkyl-1-aryl-pyrazole der Formel (I) insektizide Wirkung besitzen.

Überraschenderweise zeigen die erfindungsgemäßen 5-Amino-3-halogenalkyl-1-aryl-pyrazole der allgemeinen Formel (I) eine bessere insektizide Wirksamkeit, als die aus dem Stand der Technik bekannten 5-Amino-1-aryl-pyrazole, wie beispielsweise das 4-Cyano-5-propionamido-1-(2,4,6-trichlorphenyl)-pyrazol oder das 4-Cyano-5-propionamido-1-(2,3,4-trichlorphenyl)-pyrazol, welches chemisch naheliegende Verbindungen sind.

Die erfindungsgemäßen 5-Amino-3-halogenalkyl-1-aryl-pyrazole sind durch die Formel (I) allgemein definiert. Bevorzugt sind Verbindungen der Formel (I), bei welchen

$R^1$ für geradkettiges oder verzweigtes Halogenalkyl mit 1 bis 4 Kohlenstoffatomen und 1 bis 9 gleichen oder verschiedenen Halogenatomen steht,

$R^2$ für Cyano, für Hydroxycarbonyl, für Carbamoyl, Thiocarbamoyl, für jeweils geradkettiges oder verzweigtes Alkoxycarbonyl, N-Alkylcarbamoyl oder N,N-Dialkylcarbamoyl mit jeweils 1 bis 4 Kohlenstoffatomen in den einzelnen Alkylteilen, für jeweils geradkettiges oder verzweigtes Alkenyloxycarbonyl, N-Alkenylcarbamoyl oder N,N-Dialkenylcarbamoyl mit jeweils 3 bis 6 Kohlenstoffatomen in den einzelnen Alkenylteilen, für jeweils geradkettiges oder verzweigtes Alkinyloxycarbonyl, N-Alkinylcarbamoyl oder N,N-Dialkinylcarbamoyl mit jeweils 3 bis 6 Kohlenstoffatomen in den einzelnen Alkinylteilen oder für N-Phenylcarbamoyl steht und

Ar für einfach oder mehrfach, gleich oder verschieden substituiertes Phenyl mit Ausnahme des 4-Nitrophenylrestes steht, wobei als Substituenten infrage kommen: Halogen, Cyano, Nitro sowie jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl oder Alkoxycarbonyl mit jeweils 1 bis 4 Kohlenstoffatomen in den einzelnen Alkylteilen und gegebenenfalls 1 bis 9 gleichen oder verschiedenen Halogenatomen.

Besonders bevorzugt sind Verbindungen der Formel (I), bei welchen

$R^1$ für Trifluormethyl, Dichlorfluormethyl, Difluorchlormethyl, Difluormethyl, Trifluorethyl, Trifluorchlorethyl, Trifluordichlorethyl, Difluordichlorethyl, Tetrafluorethyl, Pentafluorethyl, Heptafluorpropyl oder Nonafluorbutyl steht,

$R^2$ für Cyano, für Hydroxycarbonyl, für Carbamoyl, Thiocarbamoyl, für Methoxycarbonyl, Ethoxycarbonyl, N-Methylcarbamoyl, N,N-Dimethylcarbamoyl, N-Ethylcarbamoyl oder N,N-Diethylcarbamoyl steht und

Ar für ein-bis fünffach, gleich oder verschieden substituiertes Phenyl mit Ausnahme des 4-Nitrophenylrestes steht, wobei als Substituenten infrage kommen: Fluor, Chlor, Brom, Iod, Cyano, Nitro, Methyl, Ethyl, n-oder i-Propyl, n-, i-, s-oder t-Butyl, Methoxy, Ethoxy, Methylthio, Methylsulfinyl, Methylsulfonyl, Methoxycarbonyl, Ethoxycarbonyl, Trifluormethyl, Dichlorfluormethyl, Difluorchlormethyl, Difluormethyl, Pentafluorethyl, Tetrafluorethyl, Trifluorchlorethyl, Trifluorethyl, Difluorchlorethyl, Trifluordichlorethyl oder ein Rest -X-$R^3$, wobei X für Sauerstoff, Schwefel, Sulfinyl oder Sulfonyl steht und $R^3$ für Trifluormethyl, Dichlorfluormethyl, Difluorchlormethyl, Difluormethyl, Pentafluorethyl, Tetrafluorethyl, Trifluorchlorethyl, Trifluorethyl, Difluorchlorethyl oder Trifluordichlorethyl steht.

Im einzelnen seien außer den bei den Herstellungsbeispielen genannten Verbindungen die folgenden 5-Amino-3-halogenalkyl-1-aryl-pyrazole der allgemeinen Formel (I) genannt:

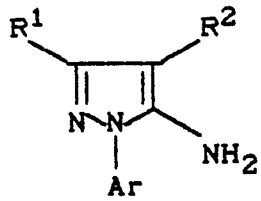

(I)

| $R^1$ | $R^2$ | Ar |
|---|---|---|
| $CF_3$ | CN | 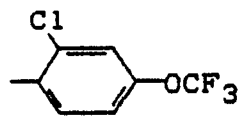 |
| $CF_3$ | CN | 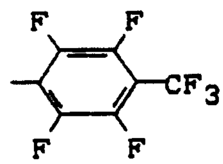 |
| $CF_3$ | $CONH_2$ | 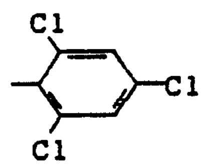 |
| $CF_3$ | $CSNH_2$ | 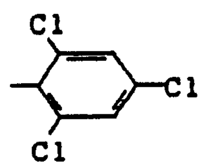 |

| $R^1$ | $R^2$ | Ar |
|---|---|---|
| $CF_3$ | $CONH_2$ | 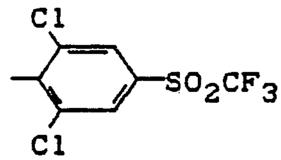 |
| $CF_3$ | $CSNH_2$ | 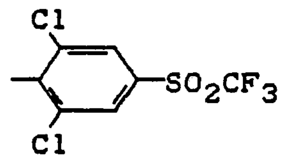 |
| $CF_3$ | $CONH_2$ | 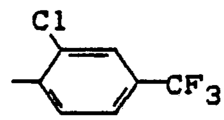 |
| $CF_3$ | $CSNH_2$ | 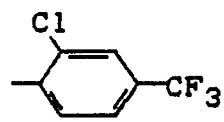 |
| $CF_3$ | $CONH_2$ | 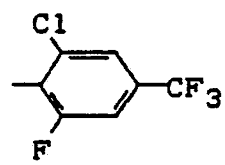 |
| $CF_3$ | $CSNH_2$ | 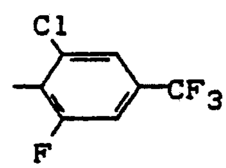 |
| $CF_3$ | $CONH_2$ | 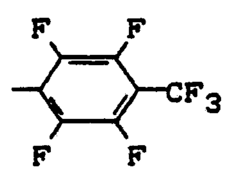 |

| $R^1$ | $R^2$ | Ar |
|---|---|---|
| $CF_3$ | $CSNH_2$ | 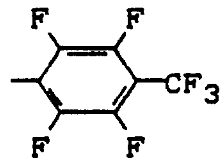 |
| $CF_3$ | $COOC_2H_5$ | 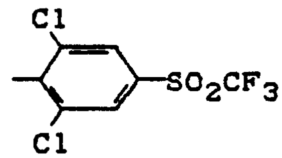 |
| $CF_3$ | COOH | 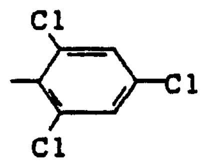 |
| $CF_3$ | COOH | 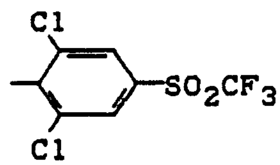 |
| $CF_3$ | COOH | 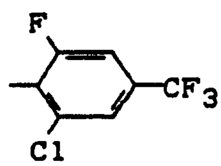 |
| $CF_3$ | $COOC_2H_5$ | 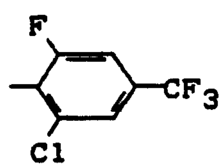 |

| $R^1$ | $R^2$ | Ar |
| --- | --- | --- |
| $CF_3$ | COOH | 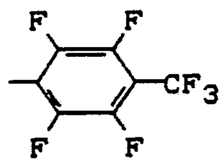 |
| $CF_3$ | $COOC_2H_5$ | 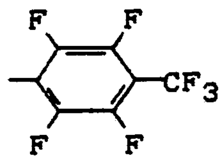 |
| $C_2F_5$ | CN | 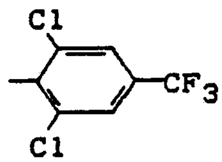 |
| $C_2F_5$ | $CONH_2$ | 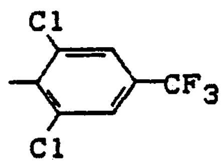 |
| $C_2F_5$ | $CSNH_2$ | 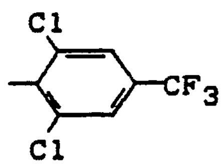 |
| $C_2F_5$ | $COOC_2H_5$ | 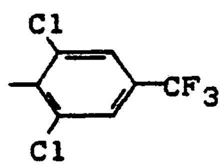 |

| $R^1$ | $R^2$ | Ar |
|---|---|---|
| $C_2F_5$ | COOH | 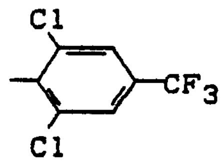 |
| $C_3F_7$ | CN | 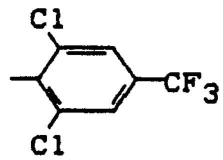 |
| $C_3F_7$ | $CONH_2$ | 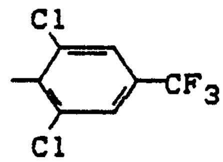 |
| $C_3F_7$ | $CSNH_2$ | 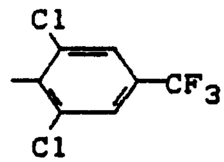 |
| $C_3F_7$ | $COOC_2H_5$ | 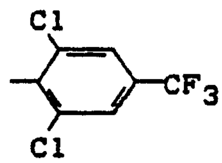 |
| $C_3F_7$ | COOH | 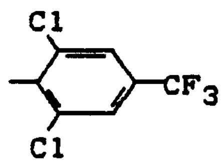 |

| $R^1$ | $R^2$ | Ar |
|---|---|---|
| $CCl_2F$ | CN | 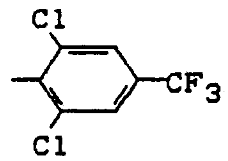 |
| $CCl_2F$ | $CONH_2$ | 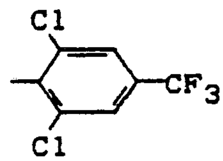 |
| $CCl_2F$ | $CSNH_2$ | 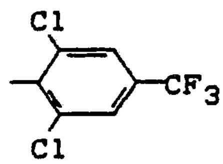 |
| $CCl_2F$ | $COOC_2H_5$ | 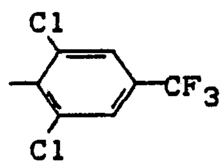 |
| $CCl_2F$ | COOH | 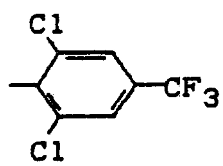 |
| $CF_3$ | $CONHCH_3$ | 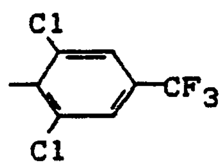 |

Verwendet man beispielsweise N-(2,2,2-Trifluor-1-brom ethyliden)-N'-(2,6-dichlor-4-trifluormethylphenyl)-hydrazin und Malodinitril als Ausgangsstoffe, so läßt sich der Reaktionsablauf des erfindungsgemäßen Verfahrens (a) durch das folgende Formelschema darstellen:

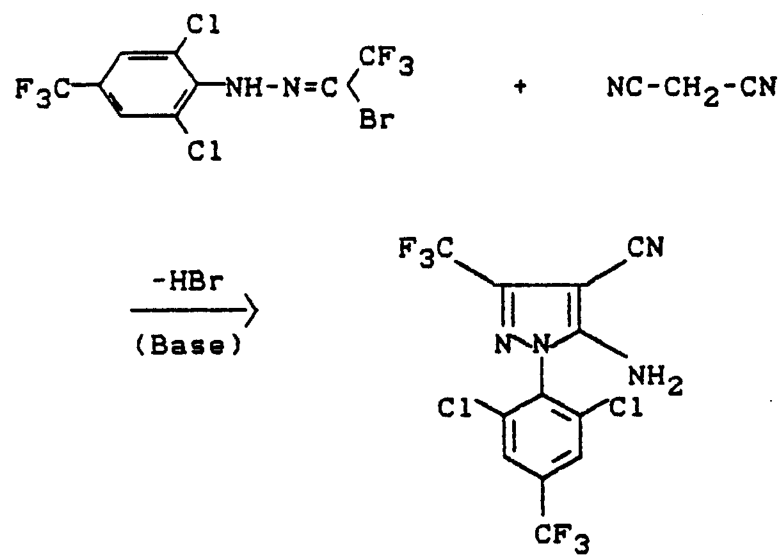


Verwendet man beispielsweise (2,2,2-Trifluor-1-chlorethyliden)-malodinitril und 2,6-Dichlor-4-trifluormethoxyphenylhydrazin als Ausgangsstoffe, so läßt sich der Reaktionsablauf des erfindungsgemäßen Verfahren (b) durch das folgende Formelschema darstellen:

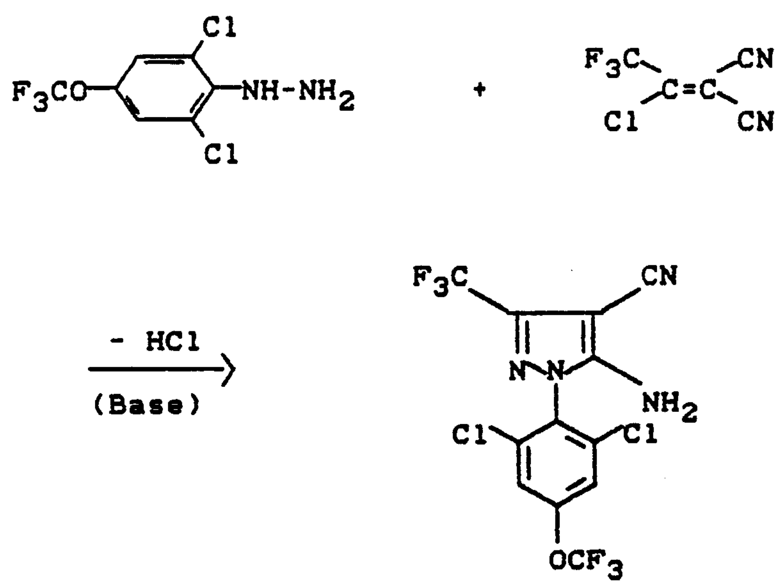


Verwendet man beispielsweise 5-Amino-4-ethoxycarbonyl-3-trifluormethyl-1-(2,4,6-trichlorphenyl)-pyrazol als Ausgangsverbindung, so läßt sich der Reaktionsablauf des erfindungsgemäßen Verfahrens (c) durch das folgende Formelschema darstellen:

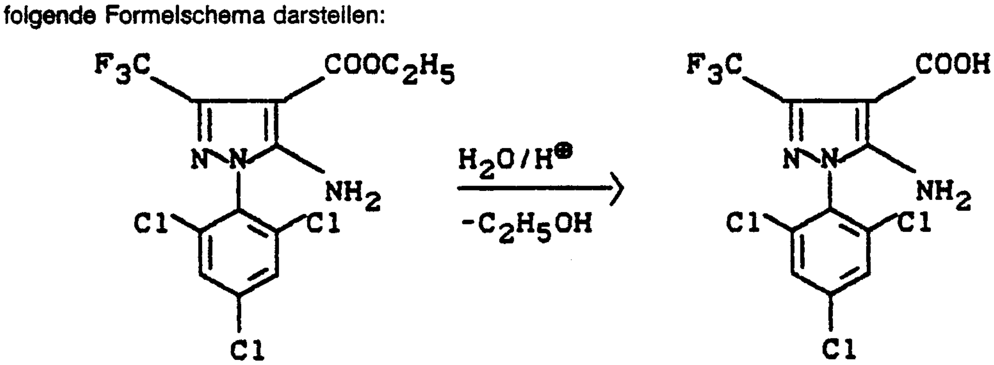

Die zur Durchführung des erfindungsgemäßen Verfahrens (a) als Ausgangsstoffe benötigten N-Arylhydrazid-halogenide sind durch die Formel (II) allgemein definiert. In dieser Formel (II) stehen $R^1$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Hal steht vorzugsweise für Chlor oder Brom.

Die N-Arylhydrazid-halogenide der Formel (II) sind teilweise bekannt (vgl. z. B. Chem. Lett. 1982, 543; J. Heterocycl. Chem. 22 565 [1985]; EP 1019).

Man erhält sie beispielsweise, wenn man Arylhydrazine der Formel (V),

$Ar\text{-}NH\text{-}NH_2$ (V)

in welcher

Ar die oben angegebene Bedeutung hat,

mit Aldehyden der Formel (VI),

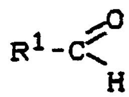 (VI)

in welcher

$R^1$ die oben angegebene Bedeutung hat,

oder mit deren Hydraten oder Halbacetalen der Formel (VII),

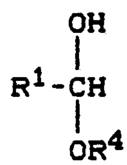 (VII)

in welcher

$R^1$ die oben angegebene Bedeutung hat und

$R^4$ für Wasserstoff oder Alkyl steht,

zunächst in einer ersten Stufe, gegebenenfalls in Gegenwart eines Verdünnungsmittels, wie beispielsweise Ethanol oder Toluol und gegebenenfalls in Gegenwart eines Reaktionshilfsmittels wie beispielsweise Schwefelsäure bei Temperaturen zwischen - 30°C + 150 °C umsetzt und die so erhältlichen Arylhydrazone der Formel (VIII),

$R^1\text{-}CH=N\text{-}NH\text{-}Ar$ (VIII)

in welcher

$R^1$ und Ar die oben angegebene Bedeutung haben,

in einer 2. Stufe mit Halogenierungsmitteln wie beispielsweise N-Bromsuccinimid, N-Chlorsuccinimid oder Brom gegebenenfalls in Gegenwart eines Verdünnungsmittels wie beispielsweise Dimethylformamid oder Essigsäure bei Temperaturen zwischen - 30 °C und + 100 °C umsetzt.

Die Aldehyde der Formel (VI), bzw. deren Hydrate oder Halbacetale der Formel (VII) sind allgemein bekannte Verbindungen der organischen Chemie (vgl. z.B. J. Am. chem. Soc., 76, 300 [1954]).

Die zur Durchführung des erfindungsgemäßen Verfahrens (a) weiterhin als Ausgangsstoffe benötigten Acetonitril-Derivate sind durch die Formel (III) allgemein definiert. In dieser Formel (III) steht $R^{2-1}$ vorzugsweise für diejenigen Reste, die bereits im Zusammenhang mit der Beschreibung der erfindungsgemäßen Stoffe der Formel (I) als bevorzugt für den Substituenten $R^2$ genannt wurden mit Ausnahme des Hydroxycarbonylrestes.

Die Acetonitril-Derivate der Formel (III) sind allgemein bekannte Verbindungen der organischen Chemie.

Die zur Durchführung des erfindungsgemäßen Verfahrens (b) als Ausgangsstoffe benötigten 2-Chloracrylnitril-Derivate sind durch die Formel (IV) allgemein definiert. In dieser Formel (IV) steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden. $R^{2-1}$ steht vorzugsweise für diejenigen Reste, die bereits im Zusammenhang mit der Beschreibung der erfindungsgemäßen Stoffe der Formel (I) als bevorzugt für den Substituenten $R^2$ genannt wurden mit Ausnahme des Hydroxycarbonylrestes.

Die 2-Chloracrylnitril-Derivate der Formel (IV) sind bekannt oder erhältlich in Analogie zu bekannten Verfahren (vgl. z.B. Zh. org. Khim. 16, 1694 [1980] bzw. C.A.: 94: 102 795 w oder Zh. org. Khim. 17, 268

[1981] bzw. C.A.: 94 174 971 y).

Die zur Durchführung des erfindungsgemäßen Verfahrens (b) und zur Synthese der Vorprodukte der Formel (II) weiterhin als Ausgangsstoffe benötigten Arylhydrazine sind durch die Formel (V) allgemein definiert. In dieser Formel (V) steht Ar vorzugsweise für diejenigen Reste, die bereits im Zusammenhang mit der Beschreibung der erfindungsgemäßen Stoffe der Formel (I) als bevorzugt für diesen Substituenten genannt wurden.

Die Arylhydrazine der Formel (V) sind bekannt (vgl. z.B. EP 154 115, EP 187 285, EP 34 945) oder erhältlich nach allgemein bekannten Verfahren in Analogie zu bekannten Verbindungen (vgl. Z.B. Houben-Weyl, "Methoden der organischen Chemie", Band X, 2, Thieme Verlag, Stuttgart 1967).

Die zur Durchführung des erfindungsgemäßen Verfahrens (c) als Ausgangsstoffe benötigten 5-Amino-3-halogen-alkyl-1-aryl-pyrazole sind durch die Formel (Ia) allgemein definiert. In dieser Formel (Ia) stehen $R^1$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

$R^{2-2}$ steht vorzugsweise für geradkettiges oder verzweigtes Alkoxycarbonyl mit 1 bis 4 Kohlenstoffatomen, insbesondere für Ethoxycarbonyl.

Die 5-Amino-3-halogenalkyl-1-aryl-pyrazole der Formel (Ia) sind erfindungsgemäße Verbindungen und erhältlich mit Hilfe der erfindungsgemäßen Verfahren (a) oder (b).

Als Verdünnungsmittel zur Durchführung des erfindungsgemäßen Verfahrens (a) kommen inerte organische Lösungsmittel infrage. Vorzugsweise verwendet man polare Lösungsmittel wie beispielsweise Dioxan, Tetrahydrofuran, Dimethylformamid, Ethanol, Methanol, t-Butanol oder Ethylenglykolmonomethylether.

Das erfindungsgemäße Verfahren (a) wird vorzugsweise in Gegenwart eines geeigneten Säurebindemittels durchgeführt. Als solche kommen alle üblicherweise verwendbaren anorganischen oder organischen Basen infrage. Vorzugsweise verwendet man Alkalimetallhydride, -hydroxide oder -alkoholate, wie beispielsweise Natriumhydrid, Natriumhydroxid, Natriummethylat, Natriumethylat, Natriumisopropylat, Kaliumisopropylat oder Kalium-t-butylat.

Die Reaktionstemperaturen können bei der Durchführung des erfindungsgemäßen Verfahrens (a) in einem größeren Bereich variiert werden. Im allgemeinen arbeitet man bei Temperaturen zwischen - 30 °C und + 150 °C, vorzugsweise bei Temperaturen zwischen 0 °C und + 100°C.

Zur Durchführung des erfindungsgemäßen Verfahrens (a) setzt man pro Mol an N-Arylhydrazidhalogenid der Formel (II) im allgemeinen 1,0 bis 10,0 Mol, Vorzugsweise 1,0 bis 2,0 Mol an Acetonitril-Derivat der Formel (III) und gegebenenfalls 1,0 bis 10,0 Mol, vorzugsweise 1,0 bis 2,0 Mol an Säurebindemittel ein. Die Reaktionsdurchführung, Aufarbeitung und Isolierung der Reaktionsprodukte erfolgt nach allgemein bekannten Verfahren (vgl. auch die Herstellungsbeispiele).

Als Verdünnungsmittel zur Durchführung des erfindungsgemäßen Verfahrens (b) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester.

Das erfindungsgemäße Verfahren (b) wird vorzugsweise in Gegenwart eines geeigneten Säurebindemittels durchgeführt. Als solche kommen alle üblichen anorganischen oder organischen Basen infrage. Vorzugsweise verwendet man tertiäre Amine wie Triethylamin, N,N-Dimethylanilin, Pyridin, Diazabicyclooctan (DABCO), Diazabicyclononen (DBN) oder Diazabicycloundecen (DBU).

Es ist auch möglich das als Reaktionspartner verwendete Arylhydrazin der Formel (V) in entsprechendem Überschuß gleichzeitig als Säurebindemittel zu verwenden.

Die Reaktionstemperaturen können bei der Durchführung des erfindungsgemäßen Verfahrens (b) in einem größeren Bereich variiert werden. Im allgemeinen arbeitet man bei Temperaturen zwischen - 30 °C und + 150°C, vorzugsweise bei Temperaturen zwischen 0 °C und + 100 °C.

Zur Durchführung des erfindungsgemäßen Verfahrens (b) setzt man pro Mol an 2-Chloracrylnitril-Derivat der Formel (IV) im allgemeinen 1,0 bis 20,0 Mol, vorzugsweise 1,0 bis 5,0 Mol an Arylhydrazin der Formel (V) und gegebenenfalls 1,0 bis 20,0 Mol, vorzugsweise 1,0 bis 2,0 Mol an Säurebindemittel ein. Die Reaktionsdurchführung, Aufarbeitung und Isolierung der Reaktionsprodukte erfolgt nach allgemein bekannten Verfahren (vgl. auch die Herstellungsbeispiele).

Das erfindungsgemäßen Verfahren (c) wird in Gegenwart einer geeigneten Säure durchgeführt. Als solche kommen alle üblicherweise verwendbaren anorganischen oder organischen Säuren infrage. Vorzugsweise verwendet man Schwefelsäure, Chlorwasserstoffsäure oder p-Toluolsulfonsäure.

Als Verdünnungsmittel zur Durchführung des erfindungsgemäßen Verfahrens (c) kommen polare anorganische oder organische Lösungsmittel oder deren Gemische mit Wasser infrage. Vorzugsweise verwendet man die gleichzeitig als Reagenz eingesetzte Schwefelsäure in entsprechendem Überschuß als Verdünnungsmittel.

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und + 150 °C, vorzugsweise bei Temperaturen zwischen + 20 °C und + 100 °C.

Zur Durchführung des erfindungesgemäßen Verfahrens (c) setzt man pro Mol an 5-Amino-3-halogenalkyl-1-aryl-pyrazol der Formel (Ia) im allgemeinen 1,0 bis 30 Mol, vorzugsweise 1,0 bis 10,0 Mol an wässriger Säure ein. Die Reaktionsdurchführung, Aufarbeitung und Isolierung der Reaktionsprodukte erfolgt nach allgemein bekannten Verfahren (vgl. auch die Herstellungsbeispiele).

Die Wirkstoffe eignen sich zur Bekämpfung von tierischen Schädlingen, vorzugsweise Arthropoden und insbesondere Insekten, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa ssp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygastr spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelisbilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia burmata, Lithocolletis blancardella, Hyponomeuta padella, Plutella maculipennis, Malacosoma neustria, Euproctis chrysorrhoea, Lymantria spp. Bucculatrix thurberiella, Phyllocnistic citrella, Agrotis spp., Euoxa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera. z.B. Diprion spp., Hoplocampa spp., Lasius spp., Monomorium pharanois, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Dabei lassen sich die erfindungsgemäßen Wirkstoffe mit besonders gutem Erfolg zur Bekämpfung von Blatt-und Bodeninsekten, wie beispielsweise gegen die Larven der Meerettichblattkäfer (Phaedon cochleariae) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Gra nulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmitteln, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolygolkol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in

weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Herstellungsbeispiele:

Beispiel 1:

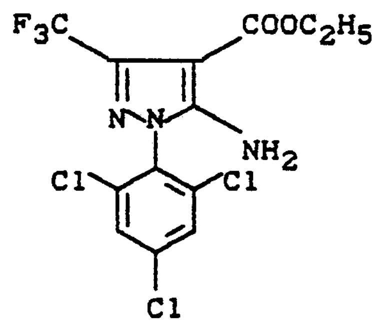

(Verfahren a)

Zu einer Lösung von 1,36 g (0,02 Mol) Natriumethanolat in 20 ml Ethanol tropft man 2,3 g (0,02 Mol) Cyanessigsäureethylester und erhitzt 15 Minuten auf Rückflußtemperatur. Anschließend tropft man 7,4 g (0,02 Mol) N-(2,4,6-Trichlorphenyl)-trifluoracethydrazid-bromid zu und kocht weitere 5 Stunden bei Rückflußtemperatur. Nach Abkühlen auf Raumtemperatur filtriert man das Natriumbromid ab und engt ein. Säulenchromatographische Auftrennung des Rückstands ergibt 4,73 g (59 % der Theorie) 5-Amino-4-ethoxycarbonyl-3-trifluormethyl-1-(2,4,6,-trichlorphenyl)-pyrazol vom Schmelzpunkt 114 - 116 °C.

Beispiel 2:

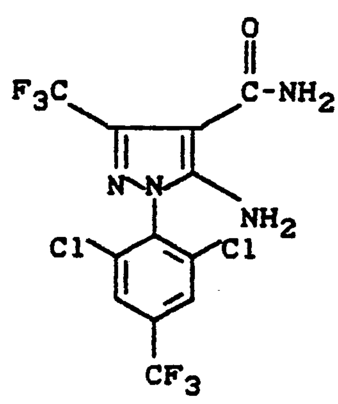

(Verfahren a)

Eine Lösung von 3,40 g (0,05 Mol) Natriumethanolat in 20 ml Ethanol versetzt man mit 4,20 g (0,05 Mol) Cyanacetamid und rührt 45 Minuten bei 25 °C nach. Nach Zutropfen von 20,2 g (0,05 Mol) N-(2,6-Dichlor-4-trifluormethylphenyl)-trifluoracethydrazid-bromid unter Kühlung rührt man 1 Stunde bei 0 °C nach. Abfiltrieren des Natriumbromids, Einengen und säulenchromatographische Reinigung liefern 1,23 g (6 % der Theorie) 5-Amino-4-aminocarbonyl-3-trifluormethyl-1-(2,6-dichlor-4-trifluormethylphenyl)pyrazol vom Schmelzpunkt 167-169 °C.

Beispiel 3:

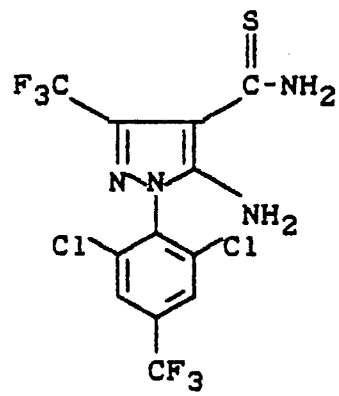

**(Verfahren a)**

Aus der Umsetzung von 3,40 g (0,05 Mol) Natriumethanolat, 5,00 g (0,05 Mol) Cyanthioacetamid und 20,2 g (0,05 Mol) N-(2,6-Dichlor-4-trifluormethylphenyl)-trifluoracethydrazidbromid in 20 ml Ethanol nach obenstehendem Verfahren isoliert man nach Säulenchromatographie 2,50 g (12 % der Theorie) 5-Amino-4-aminothiocarbonyl-3-trifluormethyl-1-(2,6-dichlor-4-trifluormethylphenyl)pyrazol vom Schmelzpunkt 123 °C - 125 °C.

Beispiel 4:

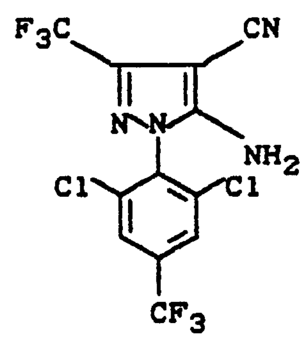

**(Verfahren b)**

Zu einer Lösung von 1,81 g (0,01 Mol) (2,2,2-Trifluor-1-chlor-ethyliden)malodinitril in 20 ml Methylenchlorid tropft man ein Gemisch von 2,45 g (0,01 Mol) 2,6-Dichlor-4-trifluormethyl-phenylhydrazin und 1,01 g (0,01 Mol) Triethylamin in 5 ml Methylenchlorid. Nach 7 Stunden Rühren bei Raumtemperatur wird eingeengt und der Rückstand säulenchromatographisch aufgetrennt. Man isoliert 2,60 g (67 % der Theorie) 5-Amino-4-cyano-3-trifluormethyl-1-(2,6-dichlor-4-trifluormethylphenyl)pyrazol vom Schmelzpunkt 196 °C - 200 °C.

Beispiel 5:

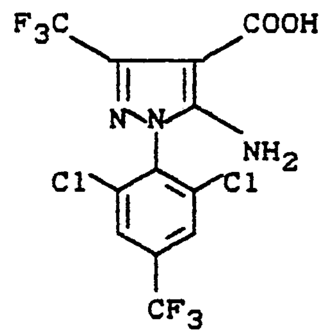

(Verfahren c)

3,05 g (0,007 Mol) 5-Amino-4-ethoxycarbonyl-3-trifluormethyl-1-(2,6-dichlor-4-trifluormethylphenyl)-pyrazol in 14 ml konzentrierter Schwefelsäure werden 5 Stunden auf 75 °C erhitzt. Nach Abkühlen auf Raumtemperatur verdünnt man mit Eiswasser und saugt ab. Man erhält 2,45 g (86 % der Theorie) 5-Amino-4-hydroxycarbonyl-3-trifluormethyl-1-(2,6-dichlor-4-trifluormethylphenyl)pyrazol vom Schmelzpunkt 191 °C - 195 °C (Zersetzung).

In entsprechender Weise und gemäß den allgemeinen Angaben zur Herstellung erhält man die folgenden 5-Amino-3-halogenalkyl-1-aryl-pyrazole der allgemeinen Formel (I):

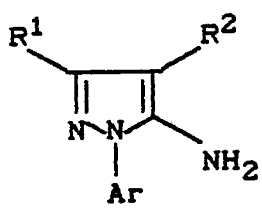

(I)

| Bsp. Nr. | $R^1$ | $R^2$ | Ar | Schmelzpunkt / °C |
|---|---|---|---|---|
| 6 | $CF_3$ | CN | 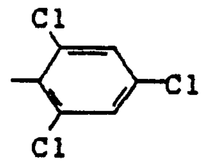 | 168-171 |
| 7 | $CF_3$ | CN | 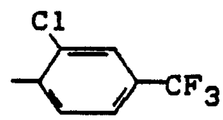 | 131-133 |
| 8 | $CF_3$ | CN | 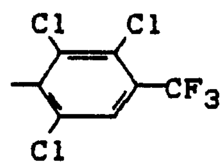 | 185-191 |
| 9 | $CF_3$ | CN | 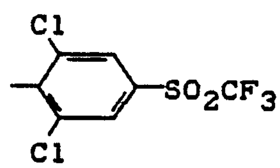 | 196-198 |
| 10 | $CF_3$ | $COOC_2H_5$ | 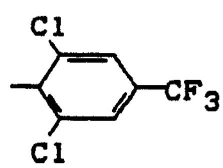 | 126-127 |

Herstellung der Ausgangsverbindungen:

Beispiel II-1:

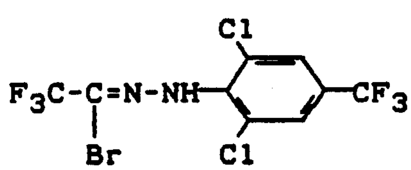

Zu einer Lösung von 8,12 g (0,025 Mol) Trifluoracetaldehyd-N-(2,6-Dichlor-4-trifluormethylphenyl)-hydrazon in 7,5 ml Dimethylformamid gibt man bei Raumtemperatur portionsweise 4,45 g (0,025 Mol) N-Bromsuccinimid, wobei eine exotherme Reaktion auftritt. Man rührt 3 Stunden bei Raumtemperatur, destilliert das Dimethylformamid ab und versetzt den Rückstand mit 20 ml Petrolether. Nach Absaugen des ausgefallenen Succinimids wird das Filtrat eingeengt und einer Kugelrohrdestillation unterworfen. Man erhält

9,26 g (91,7 % der Theorie) N-(2,6-Dichlor-4-trifluormethylphenyl)-trifluoracethydrazid-bromid vom Siedepunkt 100 °C bei 0,06 mbar und vom Brechungsindex $n_D^{20}$ 1,510.

Beispiel II-2:

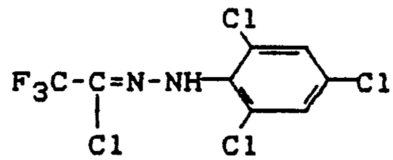

3,2 g (0,011 Mol) Trifluoracetaldehyd N-(2,4,6-Trichlorphenyl)-hydrazon und 1,5 g (0,011 Mol) N-Chlorsuccinimid in 3,3 ml Dimethylformamid werden 3 Stunden bei Raumtemperatur gerührt. Nach Einengen versetzt man mit Petrolether, filtriert und engt wieder ein. Kugelrohrdestillation des Rückstands bei 150 °C (0,5 mbar) liefert 3,28 g (89,6 % der Theorie) N-(2,4,6-Trichlorphenyl)-trifluoracethydrazid-chlorid vom Brechungsindex $n_D^{20}$ 1,549.

In entsprechender Weise und gemäß den allgemeinen Angaben zur Herstellung erhält man die folgenden N-Arylhydrazid-halogenide der allgemeinen Formel (II):

$$R^1-\underset{\displaystyle Hal}{\underset{|}{C}}=N-NH-Ar \quad (II)$$

| Bsp. Nr. | $R^1$ | Hal | Ar | physikalische Eigenschaften |
|---|---|---|---|---|
| II-3 | $CF_3$ | Br | 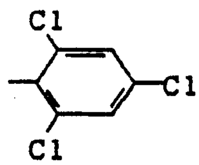 | Kp 125 °C/ 0,08 mbar |
| II-4 | $CF_3$ | Cl | 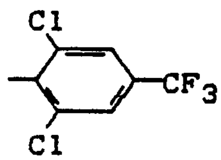 | Kp 130 °C/ 0,5 mbar |
| II-5 | $CF_3$ | Br | 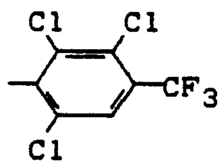 | Fp 62-63° C |
| II-6 | $CF_3$ | Br | 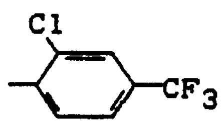 | Kp 95 °C/ 0,05 mbar |
| II-7 | $CF_3$ | Cl | 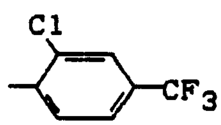 | Kp 90 °C/ 0,13 mbar |
| II-8 | $CF_3$ | Br | 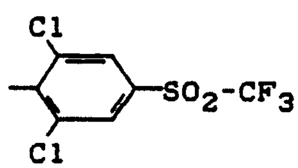 | Fp 48-50° C |

Beispiel VIII-1:

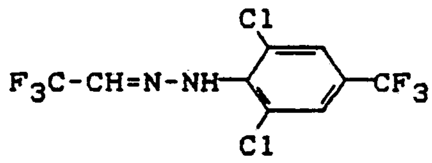

Ein Gemisch von 100 g (0,69 Mol) Trifluoracetaldehydethylhalbacetal und 169,1 g (0.69 Mol) 2,6-Dichlor-4-trifluormethylphenylhydrazin wird 6 Stunden bei 100 °C erhitzt. Nach Entfernung der flüchtigen Komponente wird der Rückstand aus Petrolether umkristallisiert. Man isoliert 200 g (89 %) Trifluoracetaldehyd N-(2,6-Dichlor-4-trifluormethylphenyl)-hydrazon vom Schmelzpunkt 45 °C bis 46 °C.

In entsprechender Weise und gemäß den allgemeinen Angaben zur Herstellung erhält man die folgenden Aldehyd-N-Arylhydrazone der allgemeinen Formel (VIII):

$R^1$-CH = N-NH-Ar (VIII)

| Bsp. Nr. | $R^1$ | Ar | Schmelzpunkt/°C |
|---|---|---|---|
| VIII-2 | $CF_3$ | 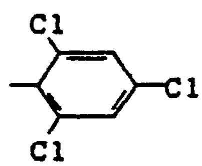 | 43-45 |

| Bsp. Nr. | $R^1$ | Ar | Schmelzpunkt/°C |
|---|---|---|---|
| VIII-3 | $CF_3$ | 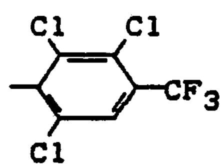 | 50-52 |
| VIII-4 | $CF_3$ | 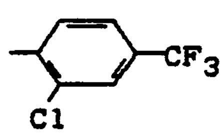 | 63-65 |
| VIII-5 | $CF_3$ | 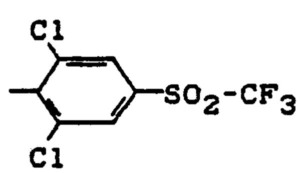 | 124-126 |

Anwendungsbeispiele:

In den folgenden Anwendungsbeispielen wurden die nachstehend aufgeführten Verbindungen als Vergleichsubstanzen (Stand der Technik) eingesetzt:

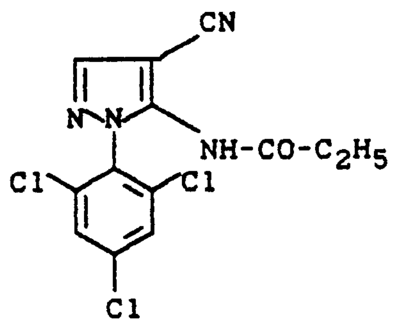

(A)

4-Cyano-5-propionamido-1-(2,4,6-trichlorphenyl)-pyrazol

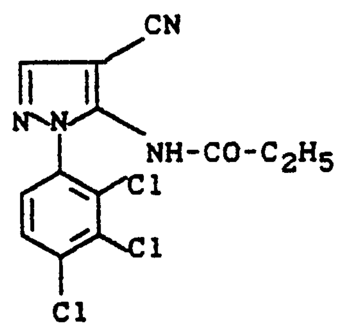

(B)

4-Cyano-5-propionamido-1-(2,3,4-trichlorphenyl)-pyrazol

(beide bekannt aus der DE-OS 32 26 513)

Beispiel A

Phaedon-Larven-Test
Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden: 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 2, 4, 6, 7, 8, 9, 10.

## Ansprüche

1. 5-Amino-3-halogenalkyl-1-aryl-pyrazole der allgemeinen Formel (I),

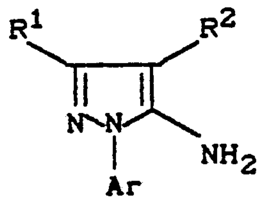

(I)

in welcher

$R^1$ für Halogenalkyl steht,

$R^2$ für Cyano, Hydroxycarbonyl, Carbamoyl, Thiocarbamoyl, Alkoxycarbonyl, N-Alkylcarbamoyl, N,N-Dialkylcarbamoyl, Alkenyloxycarbonyl, N-Alkenylcarbamoyl, N,N-Dialkenylcarbamoyl, Alkinyloxycarbonyl, N-Alkinylcarbamoyl, N,N-Dialkinylcarbamoyl oder N-Arylcarbamoyl steht und

Ar für substituiertes Phenyl mit Ausnahme des 4-Nitrophenylrestes steht.

2. 5-Amino-3-halogenalkyl-1-aryl-pyrazole gemäß Anspruch 1, Formel (I) in welcher

$R^1$ für geradkettiges oder verzweigtes Halogenalkyl mit 1 bis 4 Kohlenstoffatomen und 1 bis 9 gleichen oder verschiedenen Halogenatomen steht,

$R^2$ für Cyano, für Hydroxycarbonyl, für Carbamoyl, Thiocarbamoyl, für jeweils geradkettiges oder verzweigtes Alkoxycarbonyl, N-Alkylcarbamoyl oder N,N-Dialkylcarbamoyl mit jeweils 1 bis 4 Kohlenstoffatomen in den einzelnen Alkylteilen, für jeweils geradkettiges oder verzweigtes Alkenyloxycarbonyl, N-Alkenylcarbamoyl oder N,N-Dialkenylcarbamoyl mit jeweils 3 bis 6 Kohlenstoffatomen in den einzelnen Alkenylteilen, für jeweils geradkettiges oder verzweigtes Alkinyloxycarbonyl, N-Alkinylcarbamoyl oder N,N-Dialkinylcarbamoyl mit jeweils 3 bis 6 Kohlenstoffatomen in den einzelnen Alkinylteilen oder für N-Phenylcarbamoyl steht und

Ar für einfach oder mehrfach, gleich oder verschieden substituiertes Phenyl mit Ausnahme des 4-Nitrophenylrestes steht, wobei als Substituenten infrage kommen: Halogen, Cyano, Nitro sowie jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl oder Alkoxycarbonyl mit jeweils 1 bis 4 Kohlenstoffatomen in den einzelnen Alkylteilen und gegebenenfalls 1 bis 9 gleichen oder verschiedenen Halogenatomen.

3. 5-Amino-3-halogenalkyl-1-aryl-pyrazole gemäß Anspruch 1, Formel (I), in welcher

$R^1$ für Trifluormethyl, Dichlorfluormethyl, Difluorchlormethyl, Difluormethyl, Trifluorethyl, Trifluorchlorethyl, Trifluordichlorethyl, Difluordichlorethyl, Tetrafluorethyl, Pentafluorethyl, Heptafluorpropyl oder Nonafluorbutyl steht,

$R^2$ für Cyano, für Hydroxycarbonyl, für Carbamoyl, Thiocarbamoyl, für Methoxycarbonyl, Ethoxycarbonyl, N-Methylcarbamoyl, N,N-Dimethylcarbamoyl, N-Ethylcarbamoyl oder N,N-Diethylcarbamoyl steht und

Ar für ein-bis fünffach, gleich oder verschieden substituiertes Phenyl mit Ausnahme des 4-Nitrophenylrestes steht, wobei als Substituenten infrage kommen: Fluor, Chlor, Brom, Iod, Cyano, Nitro, Methyl, Ethyl, n-oder i-Propyl, n-, i-, s-oder t-Butyl, Methoxy, Ethoxy, Methylthio, Methylsulfinyl, Methylsulfonyl, Methoxycarbonyl, Ethoxycarbonyl, Trifluormethyl, Dichlorfluormethyl, Difluorchlormethyl, Difluormethyl, Pentafluorethyl, Tetrafluorethyl, Trifluorchlorethyl, Trifluorethyl, Difluorchlorethyl, Trifluordichlorethyl oder ein Rest $-X-R^3$, wobei X für Sauerstoff, Schwefel, Sulfinyl oder Sulfonyl steht und $R^3$ für Trifluormethyl, Dichlorfluormethyl, Difluorchlormethyl, Difluormethyl, Pentafluorethyl, Tetrafluorethyl, Trifluorchlorethyl, Trifluorethyl, Difluorchlorethyl oder Trifluordichlorethyl steht.

4. Verfahren zur Herstellung von 5-Amino-3-halogen-alkyl-1-aryl-pyrazolen der allgemeinen Formel (I),

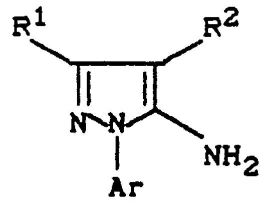

(I)

in welcher

$R^1$ für Halogenalkyl steht,

$R^2$ für Cyano, Hydroxycarbonyl, Carbamoyl, Thiocarbamoyl, Alkoxycarbonyl, N-Alkylcarbamoyl, N,N-Dialkylcarbamoyl, für Alkenyloxycarbonyl, N-Alkenylcarbamoyl, N,N-Dialkenylcarbamoyl, Alkinyloxycarbonyl, N-Alkinylcarbamoyl, N,N-Dialkinylcarbamoyl oder N-Arylcarbamoyl steht und

Ar für substituiertes Phenyl mit Ausnahme des 4-Nitrophenylrestes steht,

dadurch gekennzeichnet, daß man

(a) zum Erhalt von Verbindungen der Formel

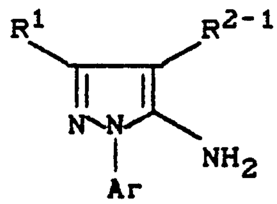

(Ia)

in welcher

$R^1$ und Ar die oben angegebene Bedeutung haben und

$R^{2-1}$ für Cyano, Carbamoyl, Thiocarbamoyl, Alkoxycarbonyl, N-Alkylcarbamoyl, N,N-Dialkylcarbamoyl, Alkenyloxycarbonyl, N-Alkenylcarbamoyl, N,N-Dialkenylcarbamoyl, Alkinyloxycarbonyl, N-Alkinylcarbamoyl, N,N-Dialkinylcarbamoyl oder für N-Arylcarbamoyl steht,

N-Arylhydrazid-halogenide der Formel (II),

$$R^1\text{-}\underset{\underset{Hal}{|}}{C}=N\text{-}NH\text{-}Ar \quad (II)$$

in welcher

$R^1$ und Ar die oben angegebene Bedeutung haben und

Hal für Halogen steht,

mit Acetonitril-Derivaten der Formel (III),

$R^{2-1}$-$CH_2$-CN (III)

in welcher

$R^{2-1}$ die oben angegebene Bedeutung besitzt,

gegebenenfalls in Gegenwart eines Verdünnungsmittels und gegebenenfalls in Gegenwart eines Säurebindemittels umsetzt, oder daß man

(b) zum Erhalt von Verbindungen der obengenannten Formel (Ia), in welcher $R^1$, $R^{2-1}$ und Ar die oben angegebene Bedeutung haben,

2-Chlor-acrylnitrilderivate der Formel (IV),

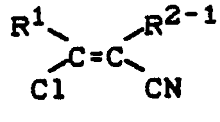

(IV)

in welcher

$R^1$ und $R^{2-1}$ die oben angegebene Bedeutung haben,

mit Arylhydrazinen der Formel (V),

Ar-NH-$NH_2$ (V)

in welcher

Ar die oben angegebene Bedeutung hat,

gegebenenfalls in Gegenwart eines Verdünnungsmittels und gegebenenfalls in Gegenwart eines

Säurebindemittels umsetzt,
oder daß man
(c) zum Erhalt von Verbindungen der Formel

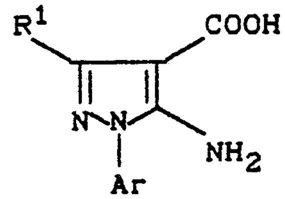

(Ib)

in welcher $R^1$ und Ar die oben angegebene Bedeutung besitzen,
die mit Hilfe der Verfahren (a) oder (b) erhältlichen 5-Amino-3-halogenalkyl-1-aryl-pyrazole der Formel (Ic),

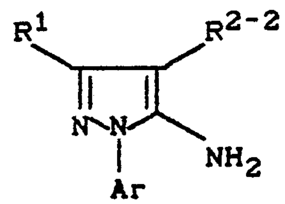

(Ic)

in welcher
$R^1$ und Ar die oben angegebene Bedeutung haben und
$R^{2-2}$ für Alkoxycarbonyl, Alkenyloxycarbonyl oder Alkinyloxycarbonyl steht,
mit Säuren gegebenenfalls in Gegenwart eines Verdünnungsmittels verseift.

5. Insektizide Mittel, gekennzeichnet durch einen Gehalt an mindestens einem 5-Amino-3-halogenalkyl-1-aryl-pyrazol der Formel (I).

6. Verfahren zur Bekämpfung von Insekten, dadurch gekennzeichnet, daß man 5-Amino-3-halogenalkyl-1-aryl-pyrazole der Formel (I) auf Insekten und/oder ihren Lebensraum einwirken läßt.

7. Verwendung von 5-Amino-3-halogenalkyl-1-aryl-pyrazolen der Formel (I) zur Bekämpfung von Insekten.

8. Verfahren zur Herstellung von insektiziden Mitteln, dadurch gekennzeichnet, daß man 5-Amino-3-halogen-alkyl-1-aryl-pyrazole der Formel (I) mit Streckmitteln und/oder oberflächenaktiven Mitteln vermischt.

Europäisches Patentamt

# EUROPÄISCHER RECHERCHENBERICHT

Nummer der Anmeldung

EP 88102602.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| --- | --- | --- | --- |
| P,A | DE - A1 - 3 603 291 (BAYER AG) * Zusammenfassung * -- | 1,4-8 | C 07 D 231/38<br>A 01 N 43/56 |
| A | DE - A1 - 3 226 496 (MAY & BAKER) * Zusammenfassung * -- | 1,4-8 | |
| A | EP - A1 - 0 053 678 (BASF) * Zusammenfassung * ---- | 1,4-8 | |

RECHERCHIERTE SACHGEBIETE (Int Cl 4)

C 07 D 231/00

Der vorliegende Recherchenbericht wurde für alle Patentansprüche erstellt.

| Recherchenort | Abschlußdatum der Recherche | Prüfer |
| --- | --- | --- |
| WIEN | 28-04-1988 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82